# EUROPEAN PATENT APPLICATION

(11) **EP 1 723 896 A1**
(43) Date of publication of application: **22.11.2006**
(21) Application number: 05709625.7
(22) Date of filing: 02.02.2005
(51) Int. Cl.: A61B 1/00

(54) **DEVICE BEING INTRODUCED INTO SUBJECT BODY**

(30) Priority: 12.03.2004 JP 2004071582
(71) Applicant: Olympus Corporation, Tokyo 151-0072 (JP)
(72) Inventor: HIRAKAWA, Katsumi, 2291103 (JP); KIMOTO, Seiichiro, 1920045 (JP); NAGASE, Ayako, 1920045 (JP); SASAGAWA, Katsuyoshi, 1910031 (JP); SUZUKI, Katsuya, 2291103 (JP); HONDA, Takemitsu, 1910062 (JP); NAKATSUCHI, Kazutaka, 1910062 (JP)
(74) Representative: von Hellfeld, Axel
(86) International application number: PCT/JP2005/001505
(87) International publication number: WO 2005/087079

(57) **Abstract**

A body-insertable apparatus includes a permanent magnet (22) covered by a biocompatible material (23), e.g., ceramic, inside a capsule-like shell (21). At the same time, a space between the shell (21) and the permanent magnet (22) is filled with a filling material (24) made of a material, such as barium sulfate, that does not adversely affect the subject body (1). An intensity of a constant magnetic field that is generated from the permanent magnet (22) and is output from the body-insertable apparatus inside the subject body (1) is detected outside the subject body (1). Thus, the simply-structured body-insertable apparatus allows for a safe acquisition of positional information thereof without a negative influence on the subject body even when the shell is decomposed.

## Description

### TECHNICAL FIELD

The present invention relates to a body-insertable apparatus that is inserted into a subject body and travels through the subject body in order to acquire positional information on a subject body interior from outside.

### BACKGROUND ART

In a field of endoscope, a capsule endoscope that has an imaging function and a radio transmission function comes along in recent years. The capsule endoscope travels through organs (through body cavity) such as a gaster and a small intestine while following peristaltic motion of the organs as well as sequentially imaging the organs using the imaging function during an observation period until the capsule endoscope is naturally discharged from a biological body (human body) of a patient after the capsule endoscope is swallowed by the patient, for an observation (examination).

Image data captured inside the body cavity by the capsule endoscope is transmitted to an external device that is provided outside the subject body by the radio transmission function such as a sequential wireless communication, during the observation period when the capsule endoscope travels through the organs. Then, the image data is stored in a memory that is provided inside the external device. The patient can freely move without any problems during the observation period from when the capsule endoscope is swallowed until when the capsule endoscope is discharged, since the patient carries around the external device that has the radio transmission function and a memory function. After the observation, a diagnosis can be made by a doctor or a nurse by displaying the image inside the body cavity on a display unit such as a display, based on the image data that are stored in the memory of the external device.

In the body cavity, a stricture, a narrowing, and a stoppage of a tract sometimes occur due to mutation of the tract towards disease. Hence, it is necessary in the observation described above to examine whether the capsule endoscope smoothly travels through the body cavity or not. Under such circumstance, it becomes beneficial to preliminarily acquire information (positional information) related to the traveling of an examination device (body-insertable apparatus) through the body cavity in order to decide whether the capsule endoscope can safely be used on the patient or not.

Therefore, conventionally, the smooth traveling of the body-insertable apparatus through the body cavity is verified by providing an electronic ID tag to the body-insertable apparatus and by electrically transmitting the positional information on the subject body interior at which the body-insertable apparatus is located (see Patent Document 1). According to the conventional technique, the body-insertable apparatus is provided with an electric power source in order to drive the electronic ID tag, which is provided for an acquisition of the positional information, and to externally transmit tag information.

Patent Document 1: International Publication No. WO 03/005877 pamphlet

### DISCLOSURE OF INVENTION

### PROBLEM TO BE SOLVED BY THE INVENTION

However, some of containers (shell) of the body-insertable apparatus, as similar to the capsule endoscope, decomposes after a certain time lapse. The shell is decomposed and there is a possibility of adversely affecting the subject body when the body-insertable apparatus which includes an electric power source stays inside the body cavity for more than a certain time due to, for example, the stricture of the tract of the body cavity.

Further, there are problems that the body-insertable apparatus has a complicated structure and the manufacturing cost is high, since devices such as the electric power source and the radio transmission device that transmits the positional information are provided in the body-insertable apparatus in chip form.

The present invention is provided in view of the foregoing, and an object of the present invention is to provide a simply-structured body-insertable apparatus that does not affect a subject body even if a container is decomposed, and that allows safe acquisition of positional information.

### MEANS FOR-SOLVING PROBLEM

In order to alleviate the above inconveniences and to achieve the object, a body-insertable apparatus according to the present invention that is inserted into a subject body and travels through the subject body includes a magnetic field generator that generates a constant magnetic field; a covering member that covers the magnetic field generator and that is made of a biocompatible material; and a container that houses and seals the magnetic field generator covered by the covering member.

In the body-insertable apparatus recited in claim 2 according to the above invention, the container is decomposed and configured to release the magnetic field generator housed therein after a predetermined time lapse from when the container is inserted into the subject body.

The body-insertable apparatus recited in claim 3 according to the above invention further includes a filler that fills the container.

In the body-insertable apparatus recited in claim 4 according to the above invention, the biocompatible material is ceramic or titanium.

In the body-insertable apparatus recited in claim 5 according to the above invention, the filler is made of normal saline or barium.

### EFFECT OF THE INVENTION

A body-insertable apparatus according to the present invention includes a magnetic field generator that outputs constant magnetic field outward from a subject body, and the magnetic field generator is covered by a biocompatible material. Consequently, decomposition of a container of the body-insertable apparatus does not affect the subject body since the magnetic field generator is covered by the biocompatible material. Therefore, the body-insertable apparatus has a simple configuration, and positional information can safely be obtained.

### BRIEF DESCRIPTION OF DRAWINGS

FIG. 1 is a diagram of a schematic configuration of a body-insertable apparatus according to the present invention;
FIG. 2 is a diagram of another schematic configuration of the body-insertable apparatus according to the present invention;
FIG. 3 is a diagram of an overall configuration of an intra-subject travel state detecting system in a subject body; and
FIG. 4 is a diagram of a configuration of a travel state deriving device shown in FIG. 3.

### EXPLANATIONS OF LETTERS OR NUMERALS

- 1: Subject body
- 2: Travel state confirming capsule
- 3: Travel state detector
- 4: Display device
- 5: Portable recording medium
- 6a - 6h: Magnetic field detector
- 7a, 7b: Securing member
- 8: Travel state deriving device
- 9: Receiving antenna
- 10: Transmitting antenna
- 21: Shell
- 22: Permanent magnet
- 23: Biocompatible material
- 24: Filling material
- 81: Reference detector selector
- 82: Selector
- 83: Distance calculator
- 84: Position calculator
- 85: Travel state information generator
- 86: Storage unit

### BEST MODE(S) FOR CARRYING OUT THE INVENTION

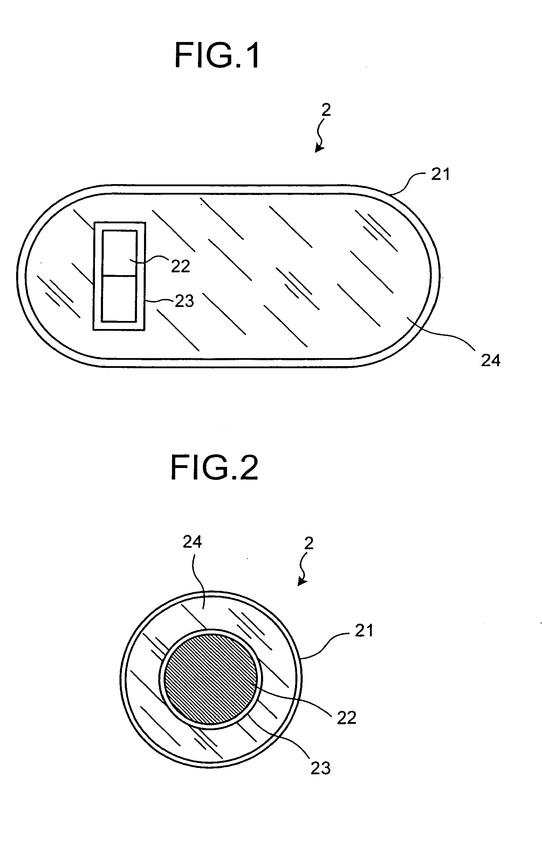

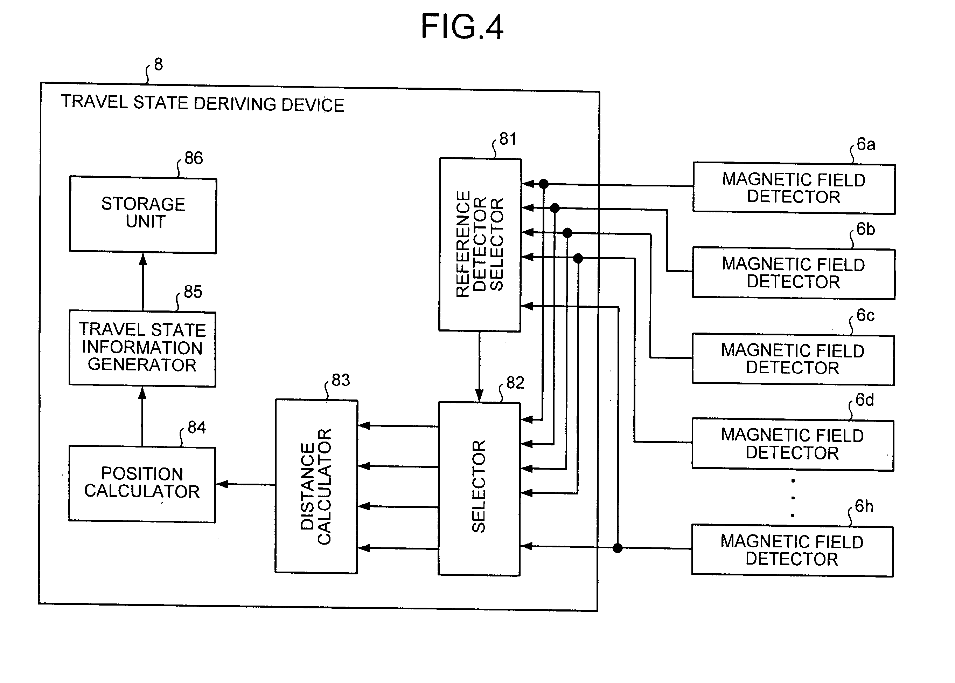

Exemplary embodiments of a body-insertable apparatus according to the present invention will be explained in details below with reference to FIGS. 1 to 4. Note that the present invention is not limited to the exemplary embodiments, and various modifications may be made without departing from the spirit or scope of the general inventive concept of the present invention.

### Embodiment 1

FIG. 1 is a diagram of a schematic configuration of a body-insertable apparatus according to the present invention, and FIG. 2 is a diagram of another schematic configuration of the body-insertable apparatus according to the present invention. The body-insertable apparatus is related to a travel state confirming capsule that is used during a preliminary examination. The preliminary examination examines whether there exists a stricture, through which it is difficult for an capsule endoscope to travel, inside a subject body or not.

In FIG. 1, a travel state confirming capsule 2 being the body-insertable apparatus includes a shell 21 that is a container and has a capsule shape similar to a shape of a container of the capsule endoscope, a permanent magnet 22 that is a magnetic field generator, coated by a biocompatible material 23, and is arranged inside the shell 21, and a filling material 24 which is a filler and fills a space between the shell 21 and the permanent magnet 22.

The shell 21 includes two distal end portions both having substantially semi-spherical dorm-like shapes and a cylindrical body portion formed between the distal end portions. The two distal end portions and the body portion integrally form the shell 21. The distal end portions and the body portion are formed of flexible material that is transformable when pressure greater than a predetermined amount is applied. The flexible material is, for example, soft gelatin or biodegradable polymer. The distal end portions and the body portion have a property of being decomposed through reaction with body fluid (especially digestive fluid of alimentary canal) that is secreted inside a subject body 1, when the travel state confirming capsule 2 stays inside the subject body 1 for a certain period. Hence, there is an advantage that does not require to perform abdominal surgery on the subject body 1 in the event that the travel state confirming capsule 2 that is inserted into the subject body 1 cannot be discharged outside of the subject body 1. Here, a time required for the shell 21 to be decomposed is determined by adjusting a thickness thereof or by laminating a material of the shell 21.

The permanent magnet 22 has a disc shape and forms constant magnetic field outside. The permanent magnet 22 is formed of, for example, samarium cobalt or neodymium. An exterior surface of the permanent magnet 22 is coated by biocompatible material that does not adversely affect the subject body 1, for example, the biocompatible material 23 such as ceramic, and titanium. The biocompatible material 23 is formed so that the biocompatible material 23 does not affect magnetic field intensity and direction of the constant magnetic field that is output from the permanent magnet 22. An outside diameter of the permanent magnet 22 is formed to be smaller than an inside diameter of the body portion of the shell 21. Further, a length of the permanent magnet 22 is formed to be shorter than a length inside the shell 21. The permanent magnet 22 is housed inside the shell 21 with the filling material 24. The permanent magnet 22 may be covered by a heat shrinkable tube whose exterior surface is coated by the biocompatible material, instead of being directly coated by the biocompatible material. Furthermore, the permanent magnet 22 may be covered as described above, so that a substantially spherical structure having the permanent magnet 22 therein is formed, and the structure may be housed in the shell 21 with the filling material 24.

The space between the shell 21 and the permanent magnet 22 is filled with the filling material 24 to prevent a movement of the permanent magnet 22 that is arranged inside the shell 21. The filling material 24 is made of, for example, normal saline or barium sulfate so that the filling material 24 does not adversely affect the subject body 1 after the shell 21 is decomposed. Particularly, the filling material 24 may be employed as a contrast agent when the filling material 24 is formed of the barium sulfate. Consequently, there is an advantage that a position of the permanent magnet 22 can be detected by, for example, X-ray inspection.

As described above, the body-insertable apparatus according to the present embodiment has the permanent magnet, which is covered by the biocompatible material, inside the shell. Therefore, even when the body-insertable apparatus that is inserted into the subject body is trapped inside the body cavity and resists discharging motion, the shell is decomposed after a certain time lapse to leave only the permanent magnet whose external surface is coated or covered with the biocompatible material, and the permanent magnet has a small diameter and a discoidal exterior shape. Consequently, the body-insertable apparatus comes to easily be discharged outside through the body cavity without adversely affecting the subject body. Hence, the body-insertable apparatus has a simple configuration and can safely acquire the positional information.

The shapes of the shell 21 and the permanent magnet 22 are not limited to the shapes described above, and the shell 21 and the permanent magnet 22 can be formed to have a substantially spherical shape as shown in FIG. 2. Here, by setting the outside diameter of the shell 21 as the same as the outside diameter of the capsule endoscope, an effect similar to the effect obtained from the configuration of FIG. 1 can be obtained.

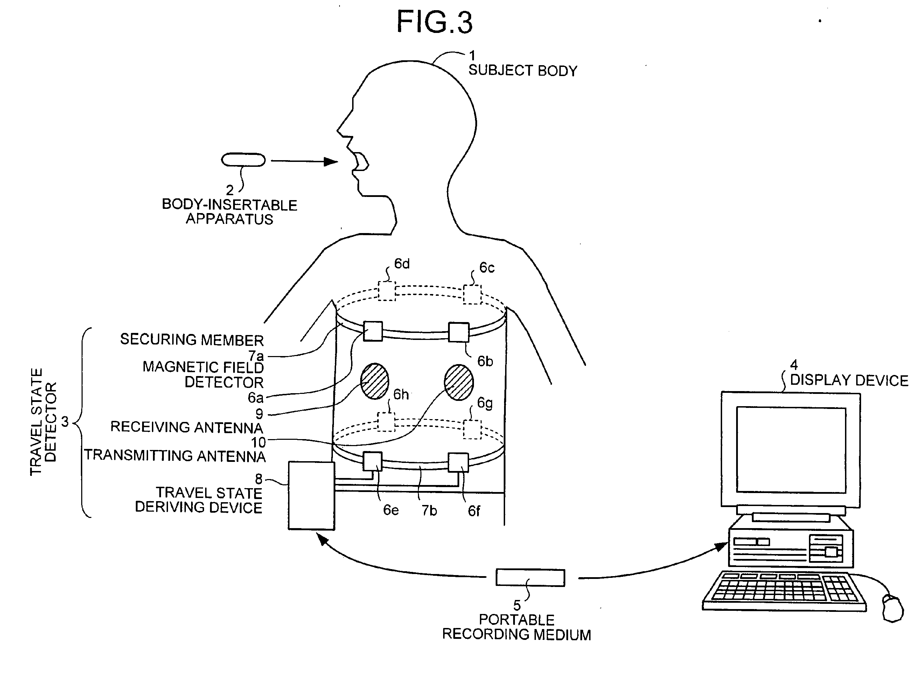

Next, an intra-subject travel state detecting system of the present embodiment is explained with reference to a schematic diagram of FIG. 3. In FIG. 3, the intra-subject travel state detecting system has the travel state confirming capsule 2 and a travel state detector 3. The travel state confirming capsule 2 is inserted into the subject body 1 and functions as an example of the body-insertable apparatus. The travel state detector 3 performs detection and the like of the travel state of the travel state confirming capsule 2 inside the subject body 1. In addition, in FIG. 3, the intra-subject travel state detecting system includes a display device 4 that displays an image and the like inside the subject body 1 obtained by the capsule endoscope, and a portable recording medium 5 that exchanges information between the travel state detector 3 and the display device 4. In brief, the intra-subject travel state detecting system of the present embodiment examines how the travel state confirming capsule travels through the subject body.

The travel state detector 3 detects the travel state of the travel state confirming capsule 2 inside the subject body 1 based on the constant magnetic field that is output from the travel state confirming capsule 2. Specifically, as shown in FIG. 3, the travel state detector 3 includes magnetic field detectors 6a to 6h, securing members 7a and 7b, and a travel state deriving device 8. The magnetic field detectors 6a to 6h detect the constant magnetic field that is output from the travel state confirming capsule 2. The securing member 7a secures the magnetic field detectors 6a to 6d to the subject body 1. The securing member 7b secures the magnetic field detectors 6e to 6h to the subject body 1. The travel state deriving device 8 calculates the position of the travel state confirming capsule 2 based on the magnetic field intensity that is detected by the magnetic field detectors 6a to 6h. In addition, the travel state detector 3 has a receiving antenna 9 that receives radio transmission signals that are transmitted from the capsule endoscope, and a transmitting antenna 10 that transmits the radio transmission signals to the capsule endoscope. The magnetic field detectors 6a to 6h, the receiving antenna 9, and the transmitting antenna 10 are electrically connected to the travel state deriving device 8, and input or output information with respect to the travel state deriving device 8. The magnetic field detectors 6a to 6h include, for example, a magneto impedance (MI) sensor, and the magnetic field detectors 6a to 6h detect the magnetic field intensity and a direction of the magnetic field at a position where each of the magnetic field detectors 6a to 6h is arranged. In the present embodiment, eight sensors are arranged so that the sensors are arranged at respective corners of a cube.

The display device 4 displays the image inside the body cavity that is picked up by the capsule endoscope not shown, and the display device 4 has a configuration as a workstation that displays the image based on data acquired from the portable recording medium 5. Specifically, the display device 4 may display the image directly through a cathode ray tube (CRT) display, a liquid crystal display, and the like, or the display device 4 may have a configuration that outputs the image to a specific medium, such as a printer.

The portable recording medium 5 is connectable to the travel state deriving device 8 and the display device 4. The portable recording medium 5 is capable of outputting and recording information when inserted, attached, and connected to the travel state deriving device 8 and the display device 4. In the present embodiment, while the travel state confirming capsule 2 is traveling through the body cavity of the subject body 1, the portable recording medium 5 is attached and inserted into the travel state deriving device 8 and records information related to the position of the travel state confirming capsule 2. After the travel state confirming capsule 2 is discharged from the subject body 1, the portable recording medium 5 is removed from the travel state deriving device 8 and is attached and inserted to the display device 4. The display device 4 reads out the data recorded in the portable recording medium 5. The portable recording medium 5 is, for example, a Compact Flash (registered trademark) memory so that the data input-output between the travel state deriving device 8 and the display device 4 can be performed indirectly through the portable recording medium 5. Hence, the subject body 1 can freely move even if the travel state confirming capsule 2 is traveling through the subject body 1, which is different from a system in which the travel state deriving device 8 and the display device 4 are directly connected to each other by a cable.

The travel state deriving device 8 includes a reference detector selector 81, a selector 82, a distance calculator 83, a position calculator 84, and a travel state information generator 85. The reference detector selector 81 selects a reference detector from the magnetic field detectors 6a to 6h. The selector 82 selects selected devices based on the selected reference detector, and outputs the magnetic field intensity that is acquired by the reference detector and the referenced devices. The distance calculator 83 calculates a distance to the travel state confirming capsule 2 based on the magnetic field intensity that is output from the selector 82. The position calculator 84 calculates a position of the travel state confirming capsule 2 based on the calculated distance. The travel state information generator 85 generates travel state information based on the calculated position. Further, the travel state deriving device 8 includes a storage unit 86 that stores the travel state information that is generated by the travel state information generator 85. Then, the configuration in which the travel state information is output to the display device 4 through the storage unit 86 and the portable recording medium 5 allows a doctor and the like to grasp the travel state of the travel state confirming capsule 2.

In the present embodiment, the travel state information to be derived is variation in position of the travel state confirming capsule 2. However, the travel state information to be derived is not limited to the variation in position, and may include variation in orientation direction, i.e., an orientation of the travel state confirming capsule 2. The variation in the orientation, i.e., a pointed direction of a longitudinal axis of the travel state confirming capsule 2, may be derived according to a positional dependence of the travel direction of the constant magnetic field output from the permanent magnet 22. Further, the travel state confirming capsule 2 and the permanent magnet 22 can be formed spherically. Consequently, the insertion of the body-insertable apparatus into the subject body and the discharging thereof to outside of the subject body 1 become easier.

The travel state confirming capsule is described above as the body-insertable apparatus in the present embodiment. However, the present invention is not limited to use of the travel state confirming capsule as the body-insertable apparatus, and the configuration of the body-insertable apparatus according to the present invention can be employed for the capsule endoscope. Then, the image information inside the subject body and the positional information inside the subject body can be obtained simultaneously, whereby an imaging position can be assumed easily.

### INDUSTRIAL APPLICABILITY

A body-insertable apparatus according to the present invention is useful for a medical observation device that is inserted into a human body and observes a portion to be examined. Specifically, the body-insertable apparatus does not affect a subject body even if a shell is decomposed. The body-insertable apparatus has a simple configuration and suitable for safely obtaining positional information on the body-insertable apparatus.

## Claims

1. A body-insertable apparatus that is inserted into a subject body and travels through the subject body, comprising:
a magnetic field generator that generates a constant magnetic field;
a covering member that covers the magnetic field generator and that is made of a biocompatible material; and
a container that houses and seals the magnetic field generator covered by the covering member.

2. The body-insertable apparatus according to claim 1, wherein
the container is decomposed and configured to release the magnetic field generator housed therein after a predetermined time lapse from when the container is inserted into the subject body.

3. The body-insertable apparatus according to claims 1 or 2, further comprising
a filler that fills the container.

4. The body-insertable apparatus according to claims 1 or 2, wherein
the biocompatible material is ceramic or titanium.

5. The body-insertable apparatus according to claim 3, wherein
the filler is made of normal saline or barium.
